(19) Europäisches Patentamt
European Patent Office
Office européen des brevets

(11) Publication number: **0 253 010 B1**

(12) # EUROPEAN PATENT SPECIFICATION

(45) Date of publication of patent specification: **02.12.92**

(51) Int. Cl.[5]: **C07D 211/26**, C07D 211/74, C07D 211/58, C07D 211/94, C07D 241/04, C07D 241/08, C07D 295/22, C07D 295/12, C08K 5/34

(21) Application number: **86109748.3**

(22) Date of filing: **16.07.86**

The file contains technical information submitted after the application was filed and not included in this specification

(54) **N-(substituted cyclic alkyleneimine)-alpha-(3,5-di-alkyl-4-hydroxy-phenyl)-alpha, alpha-dialkyl acetamides.**

(43) Date of publication of application:
**20.01.88 Bulletin 88/03**

(45) Publication of the grant of the patent:
**02.12.92 Bulletin 92/49**

(84) Designated Contracting States:
**AT BE CH DE FR GB IT LI LU NL SE**

(56) References cited:
**EP-A- 0 019 237**
**EP-A- 0 104 645**
**EP-A- 0 112 802**
**EP-A- 0 184 154**
**DE-A- 2 647 452**
**EP-A- 0 082 817**
**EP-A- 0 111 448**
**EP-A- 0 141 419**
**DE-A- 2 621 870**
**US-A- 4 780 495**

(73) Proprietor: **The B.F. GOODRICH Company**
**Dept. 0015 WHB-6 500 South Main Street**
**Akron, Ohio 44318(US)**

(72) Inventor: **Lai, John Ta-Yuan**
**3465 Ridge Park Blvd.**
**Broadview Heights, Ohio 44147(US)**
Inventor: **Son, Pyong Nae**
**2106 Ayers Avenue**
**Akron, Ohio 44313(US)**

(74) Representative: **von Kreisler, Alek, Dipl.-Chem. et al**
**Deichmannhaus am Hauptbahnhof**
**W-5000 Köln 1(DE)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

### SUMMARY OF THE INVENTION

Novel N-(substituted)-1-(piperazine alkyl) or -4-(piperidine)-α-(3,5-di-alkyl-4-hydroxyphenyl)-α,α-dialkyl acetamides prepared by a new process are novel stabilizers for organic materials subject to attack and degradation by heat, oxygen and light, and form useful combinations for this purpose with other hindered phenol stabilizers.

Documents EP-A-0112802, EP-A-0082817, EP-A-0141419, EP-A-0111448, EP-A-0104645 and EP-A-0184154 also disclose 3,5-dialkyl-4-hydroxyphenyl-alkylcarbonylamido-4-(2,2,6,6-tetraalkyl)piperidines which exhibit stabilizing properties against the degradation of organic material by heat, oxygen and light.
In detail EP-A-0112802 discloses compounds bearing a $-C(R_3R_4)-CH_2-$ radical between the phenol-moiety and the carbonyl-group. EP-A-0082817 and EP-A-0111448 disclose compounds bearing a $-(CH_2)_n-$ radical in said position between the phenol-moiety and the carbonyl-group.
From EP-A-0141419, EP-A-0104645 and EP-A-0184154 it is known that not only 3,5-dialkyl-4-hydroxyphenyl-alkylcarbonylamido but also the 3,3,5,5-tetraalkyl-piperazine-2-one moiety constitute a basic structure for stabilizing properties.

### DETAILED DESCRIPTION

The novel N-(substituted)-1-(piperazine alkyl) or -4-(piperidine)-α-(3,5-di-alkyl-4-hydroxyphenyl)-α,α-dialkyl acetamides having the general formula

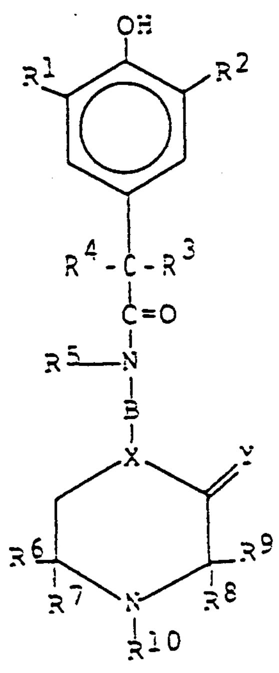

wherein $R^1$ and $R^2$ are alkyl and cycloalkyl groups containing up to 12 carbon atoms, alkylcycloalkyl groups wherein the alkyl groups contain 1 to 8 carbon atoms; $R^3$ and $R^4$ are alkyl groups containing 1 to 18 carbon atoms, $R^5$ is hydrogen, alkyl and cycloalkyl containing up to 12 carbon atoms, alkyl-cycloalkyl and aryl or alkaryl groups wherein the alkyl groups contain 1 to 8 carbon atoms, $R^6$, $R^7$, $R^8$ and $R^9$ are alkyl and cycloalkyl groups containing up to 12 carbon atoms, alkylcycloalkyl and aryl or alkaryl groups wherein the alkyl groups contain 1 to 8 carbon atoms, B is a bond in case X represents a -CH-group or in case X represents N, B is an alkylene group $-(CH_2)_n-$ wherein n is 1 to 8; $R^{10}$ is hydrogen or alkyl radicals containing 1 to 18 carbon atoms, O or OH; and Y is $H_2$ or O; whereby aryl represents phenyl or naphthyl.

Preferably $R^1$, $R^2$, $R^5$, $R^6$, $R^7$, $R^8$, $R^9$ and $R^{10}$ are alkyl groups containing 1 to 8 carbon atoms. More preferably at least one of $R^1$ or $R^2$ is a t-alkyl group including t-butyl and t-amyl; $R^3$ and $R^4$ are alkyl groups containing 1 to 8 carbon atoms, and more preferably 1 to 4 carbon atoms; $R^5$ is an alkyl group containing 1 to 6 carbon atoms; $R^6$ and $R^7$ are methyl; $R^8$ is an alkyl group containing 1 to 3 carbon atoms; $R^9$ is an

alkyl group containing 1 to 4 carbon atoms; $R^{10}$ is hydrogen; in B n is 2 to 4.

These N-(substituted)-1-(piperazine alkyl) or -4-(piperidine)-α-(3,5-di-alkyl-4-hydroxyphenyl)-α,α-dialkyl acetamides, more specifically the N-(2,2,6,6-tetraalkyl-4-piperidinyl)-α-(3,5-di-alkyl-4-hydroxyphenyl)-α,α-dialkylacetamides and N-alkyl-N'-(3,3,5,5-tetraalkyl-2-keto-1-piperazinealkyl)-α-(3,5-di-alkyl-4-hydroxyphenyl) -α,α-dialkylacetamides, are prepared by a new process by reacting a 2,6-di-alkylphenol with a dialkyl ketone, a haloform and a piperidine or piperazine compound in the presence of alkali metal hydroxide. An organic solvent may be used, or large amounts of the ketone reactant may be employed. The amide is isolated in excellent yields by crystallizing the filtered reaction product.

Typical 2,6-dialkyl phenols used have the formula

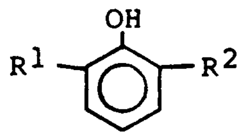

wherein $R^1$ and $R^2$ have the meanings set forth above, including 2-methyl-6-t-butylphenol, 2-ethyl-6-t-butylphenol, 2-propyl-6-t-butylphenol, 2-isopropyl-6-t-butylphenol, 2-n-butyl-6-t-butylphenol, 2,6-di-t-butylphenol, 2-n-amyl-6-t-butylphenol, 2-isoamyl-6-t-butylphenol, 2-hexyl-6-t-butylphenol, 2-heptyl-6-t-butylphenol, 2-isooctyl-6-t-butylphenol, 2-isopropyl-6-methylphenol, 2-n-butyl-6-isopropylphenol, 2-isoamyl-6-ethylphenol, 2-isoamyl-6-methylphenol, 2-isooctyl-6-methylphenol, 2-isooctyl-6-ethylphenol, 2-isooctyl-6-n-propylphenol or 2-isooctyl-6-n-hexylphenol.

Typical ketones that may be used in the novel process to make the new 3,5-dialkyl-4-hydroxyphenyl-substituted acetic acids of the invention are alkyl ketones of the formula

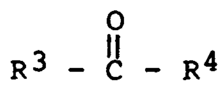

wherein $R^3$ and $R^4$ are alkyl radicals containing 1 to 18 carbon atoms, preferably 1 to 8 carbon atoms, including for example acetone, methyl ethyl ketone, methyl n-propyl ketone, diethyl ketone, 2-hexanone, 3-hexanone, di-n-propyl ketone, 2-octanone, methyl isopropyl ketone in amounts from at least 1 mole of ketone per mole of 2,6-dialkylphenol, up to amounts sufficient for the ketones to be the solvent of the reaction, 10 moles or more. No more than two moles is preferred when the ketone is a reactant only. Use of less than 1 mole reduces the product yield, and more than 2 moles is unnecessary unless the ketone is the solvent.

The haloform, such as chloroform, is also used in a molar ratio of at least one mole per mole of 2,6-dialkylphenol used, but preferably a slight molar excess is used up to a 50 percent molar excess, i.e., 1.5 mole per mole of 2,6-dialkylphenol. While larger amounts may be used, there is no advantage realized, and lesser amounts will decrease the ultimate yield of the desired product. Bromoform may be substituted for the chloroform and excellent results will be obtained.

The alkali metal hydroxide is used in powder form, or in solution, and includes sodium hydroxide and potassium hydroxide. Preferably, a molar excess of the alkali metal hydroxide is used in relation to the amount of 2,6-dialkylphenol present. Normally from 4 moles of alkali metal hydroxide per mole of 2,6-dialkylphenol up to 10 or more moles may be used, but the amount used preferably is 4 to 8 moles of hydroxide per mole of 2,6-di-alkylphenol. Use of less than 4 moles will reduce the yield of desired product.

The N-substituted cyclic alkyleneimines used in the process of this invention to make the novel acetamides, the piperidines and the piperazines, have the general formula

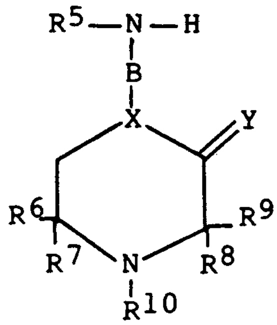

wherein $R^5$, $R^6$, $R^7$, $R^8$, $R^9$, $R^{10}$, X, Y and B are as defined above.

Typical compounds are substituted piperidine and piperazine derivatives such as 4-amino-2,2,6,6-tetramethyl piperidine, N -(2-iso-propylaminoethyl)-3,3,5,5-tetramethyl-2-piperazinone, N -(2-cyclohexylamino ethyl)-3,3,5,5-tetramethyl-2-piperazinone, 4-methylamino-2,2,6,6-tetramethyl piperidine, N-(2-isobutylaminoethyl)-3,5,5-trimethyl-3-isobutyl-2-piperazinone.

The amount of substituted piperidine or piperazine used in the reaction is based on one mole of the 2,6-dialkylphenol. While the amount may vary from less than a stoichiometric amount, resulting in low product yield, to larger excesses that are not required and have to be separated from the reaction mixture, normally 1 to 4 moles to 1 mole is used.

The solvent used may be any polar organic solvent, including an excess of the ketone used in place of an added solvent. Typical solvents that may be used include methylene chloride, tetrahydrofuran, diethyl ether, dibutyl ether, dimethyl sulfoxide, 1,4-dioxane, carbon tetrachloride, toluene and xylene.

The amounts of solvent used will vary from 5 moles to 100 moles per mole of 2,6-dialkylphenol used. As has been stated, the solvent may be eliminated if an excess of the reactant ketone is used. In this case, the amount of ketone used, based on the moles of 2,6-dialkylphenol used may be from 5 to 20, preferably 7.5 to 15 moles per mole of 2,6-dialkylphenol.

While the reactants may be added in any order, it is preferred that the alkali metal hydroxide be added last, over a period of time to control the exothermic reaction and preferably maintain the reaction below 30 ° C, preferably below 10 ° C. The reaction temperature may be varied from 0 ° C to 30 ° C, but preferably is conducted from 0 ° C to 10 ° C. The reaction time normally will vary from 5 to 15 hours.

The amides are readily isolated from the reaction mixture by filtration, washing the filtrate with aqueous inorganic acid, including hydrochloric or sulfuric acid, to the reaction mixture to isolate the amide, most of which is in the organic layer that forms. The aqueous layer may be extracted with solvent to remove all traces of the amide, and this is added to the other organic layer and this mixture is dried with a desiccant such as anhydrous sodium sulfate, and heated to dryness. This product may be recrystallized if desired.

The practice of the invention is demonstrated in the following Examples. The structures of the amides of the following Examples were confirmed by infrared and nuclear magnetic resonance spectra. Molecular weights were determined and confirmed by field desorption mass spectra (FD/MS). Elemental analysis of carbon and hydrogen was done and the amounts found were consistent with the formulas of the materials.

EXAMPLE 1

N-methyl-N -(2,2,6,6-tetramethyl-4-piperidinyl)-2-(3,5-di-t-butyl-4-hydroxyphenyl)-2-methyl propionamide

0.1 Mole of 2,6-di-t-butylphenol, 1.0 mole of acetone, 0.15 mole of chloroform and 0.1 mole of 4-methylamino-2,2,6,6-tetramethylpiperidine were added to a reactor and mixed by stirring while being cooled to 5 ° C by a circulating cold bath. 0.5 mole of powdered sodium hydroxide was slowly added in small portions over a period of 1 hour. The reaction mixture was stirred at 10 ° C overnight. The reaction mix from the reactor was filtered. The solid residue was washed with methylene chloride and the wash added to the filtrate. The filtrate was washed with 50 ml 4N hydrochloric acid, 50 ml of 5% sodium carbonate, and was then dried over sodium sulfate. The filtrate was evaporated to dryness and the dried product was washed with hexane. The resulting amide was a white solid that had a melting point of 168-173 ° C and a molecular weight of 444. By elemental analysis it was determined that the amide contained 75.38% carbon (75.67% calculated), 10.76% hydrogen (calculated 10.88%) and 6.20% nitrogen (calculated 6.30%).

EXAMPLE 2

N-Isopropyl-N -[2-(3,3,5,5-tetramethyl-1-piperazin-2-onyl)]ethyl-2-(3,5-di-t-butyl-4-hydroxyphenyl)-2-methylpropionamide

0.1 Mole of 2,6-di-t-butylphenol, 1.0 mole of acetone, 0.15 mole of chloroform, 0.1 mole of 1-(2-isopropylaminoethyl-3,3,5,5-tetramethyl-2-piperazinone were added to a reactor and mixed by stirring while being cooled by a circulating cold bath. 0.5 mole of powdered sodium hydroxide was slowly added over a period of 1 hour. The reaction mixture was stirred at 10°C overnight. The reaction mix from the reactor was filtered. The solid residue was washed with methylene chloride and the wash added to the filtrate. The filtrate was washed with 50 ml of 4N hydrochloric acid, 50 ml of 5% sodium carbonate and was then dried over sodium sulfate. The filtrate was evaporated to dryness and the dried product was washed with hexane. The resulting amide was a white solid that had a melting point of 165-169°C and a molecular weight of 515. By elemental analysis it was determined that the amide contained 72.43% carbon (72.19% calculated), 10.26% hydrogen (calculated 10.36%) and 8.02% nitrogen (calculated 8.15%).

To demonstrate the stabilizing activity of the amides of this invention, test samples of the amides in polypropylene were prepared by mixing the stabilizer compounds with polypropylene in a Brabender Plasticorder® fitted with a Cam-Head® (mixing chamber). The polypropylene is first masticated for 1 1/2 minutes at 190°C. Then the stabilizer is added, followed by 3 minutes additional mixing. The mass is removed and pressed into 508 µm (20 mil) thick sheets. From these sheets are cut 2.54 cm x 2.54 cm (1" x 1") plaques for oven aging. Type C 7.62 cm x 0.32 cm (3" x 1/8") tensil bars are cut for UV stability tests.

Thermal/oxidative stability (oven aging) testing consisted of aging the samples in triplicate in an air-circulating oven at 125°C. The time to catastrophic crumbling (failure) of the plaque was measured and reported as days to failure. Each sample contained 0.1 weight part of the named amide per 100 weight parts of polypropylene (pHr). The following results, in days to failure, were obtained:

| | 125°C |
|---|---|
| Control | 2 |
| N-methyl-N -(2,2,6,6-tetramethyl-4-piperidinyl)-2-(3,5-di-t-butyl-4-hydroxyphenyl)-2-methyl-propionamide | 7 |
| N-Isopropyl-N -[2-(3,3,5,5-tetramethyl-1-piperazin-2-onyl) ]ethyl-2-(3,5-di-t-butyl-4-hydroxyphenyl)-2-methylpropionamide | 9 |

Samples containing 0.1 weight part of the amide listed below was tested for ultraviolet light stability, i.e., resistance to degradation by UV radiation. The samples were tested in an Atlas Xenon Weatherometer®, Model No. 65-WR, equipped with a 6500 watt Xenon burner tube in accordance with ASTM #D2565-79-A. The black panel temperature was 60°C. The samples were subjected to an 18-minute water cycle every 2 hours. The time in hours to a 50% loss in tensile strength was determined. For comparison purposes a samples with no amide was tested. The following results, in hours, were obtained:

| | Hours |
|---|---|
| Blank (Control) | 220 |
| N-Isopropyl-N -[2-(3,3,5,5-tetramethyl-1-piperazin-2-onyl)ethyl]-2-(3,5-di-t-butyl-4-hydroxyphenyl)-2-methylpropionamide (0.1 phr) | 1280 |

The N-(2,2,6,6-tetraalkyl-4-piperidinyl)-3,5-di-alkyl-4-hydroxyphenyl)-α,α-dialkylacetamides and N-alkyl-N -(3,3,5,5-tetraalkyl-2-keto-1-piperazinealkyl)-α-(3,5-di-alkyl-4-hydroxyphenyl)-α,α-dialkylacetamides as defined herein provide exceptional heat stability and resistance to ultraviolet degradation to polyolefin polymers. They are especially useful for the stabilization of α-monoolefin homopolymers and copolymers, wherein the α-monoolefin contains 2 to 8 carbon atoms. High and low-density polyethylene, isotactic and atactic polypropylene, polyisobutylene, and poly(4-methyl-1-pentene) have excellent resistance to heat and oxygen when stabilized with the combinations of the present invention. Ethylene-propylene copolymers and ethylene-propylene terpolymers, generally containing less than 10 percent by weight of one or more monomers containing multiple unsaturatation provided, phenols; ring opened olefin polymers.

Polymer blends, that is, physical admixture of two or more polymers may also be stabilized in

accordance with the present invention.

In addition to polymeric materials, the present compounds may stabilize a wide variety of other organic materials. Such compounds include: waxes, synthetic and petroleum-derived lubricating oils and greases; animal oils such as, for example, fat, tallow, lard, codliver oil, sperm oil; vegetable oils such as castor, linseed, peanut, palm or cotton seed; fuel oil, diesel oil, gasoline.

The (3,5-dialkyl-4-hydroxyphenyl)-substituted amides may be used with other stabilizers including hindered or partially hindered phenols, hindered amine light stabilizers, phosphites, o-hydroxybenzophenones.

Typical hindered phenols are the hydroxyphenylalkyleneyl isocyanurates such as the symmetrical tris-(3,5-di-t-alkyl-4-hydroxybenzyl) isocyanurates; tetrakis[methylene 3-(3',5'-dialkyl-4'-hydroxyphenyl)-propanoate] methanes wherein the alkyl groups contain 1 to 8 carbon atoms, such as tetrakis[methylene 3-(3',5'-di-t-butyl-4'-hydroxyphenyl)propanoate]methane; alkyl (3-3',5'-di-t-butyl-4'-hydroxyphenyl)propionates wherein the alkyl groups contain 1 to 18 carbon atoms, such as octadecyl 3-(3',5'-di-t-butyl-4-hydroxyphenyl)propionate; 1,3,5-trimethyl-2,4,6-tris[3,5-dialkyl-4-hydroxybenzyl)benzene; 1,3,5-tris(3,5-di-t-butyl-4-hydroxyhydrocinnamoylethyl)-s-triazine-2,4,6-(1H,3H,5H)-trione; 2,2'-alkylidene bis (4,6-dialkylphenol)-s wherein the alkyl group contains 1 to 8 carbon atoms, such as 2,2'-methylene bis(4,6-di-t-butylphenol), 2,2'-ethylidene bis(4,6-di-t-butylphenol), and 2,2'-methylene bis(4-methyl-6-t-butylphenol).

For example, the following combinations provided excellent ageing in polypropylene as shown in the Weatherometer: 0.05 phr of N-isopropyl-N -[2-(3,3, 5,5-tetramethyl-1-piperazin-2-onyl)ethyl]-2-(3,5-di-t-butyl-4-hydroxyphenyl)-2-methylpropionamide, 0.05 phr of 1,1'-(1,2-ethanediyl)bis(3,3,5,5-tetramethyl piperazinone, and 0.05 phr of 2,2',2''-tris[3(3,5-di-t-butyl-4-hydroxyphenyl)propionyloxy]ethyl isocyanurate - more than 2,000 hours; 0.125 phr of N-isopropyl-N -[2-(3,3, 5,5-tetramethyl-1-piperazin-2-onyl)ethyl]-2-(3,5-di-t-butyl-4-hydroxyphenyl)-2-methyl propionamide, 0.05 phr of 2,2',2''-tris[3(3,5-di-t-butyl-4-hydroxyphenyl)-propionyloxy]ethyl isocyanurate, and 0.125 phr of 3,9-bis (octadecyloxy)-2,4,8,10-tetraoxa-3,9-diphosphaspiro[5, 5]-undecane - more than 2,000 hours.

The compounds are readily incorporated into materials to be patented by dissolving or dispersing them with the materials, in liquids, dispersions, soluations, and solid forms. If the material is a solid, especially a polymeric solid such as rubber or a plastic, the compounds can be admixed using mixers such as Banburys, extruders, two-roll mills, and the like, following conventional techniques. One way to disperse the compounds in plastic materials is to dissolve or suspend the compounds in a solvent or diluent, mix the mixture with a plastic in powder or solution form, and then evaporate the solvent.

Compositions containing the novel combination of compounds can also contain other known compounding ingredients such as fillers like carbon black, silica, metal carbonates or talc; pigments and colorants; curative ingredients like sulfur and peroxides, and vulcanization accelerators; fungicides; processing aids, reinforcing agents and standard ingredients known to the art. Other ingredients known in the art as ultraviolet light, thermal and/or oxidative stabilizers can also be used.

## Claims

### Claims for the following Contracting States : BE, CH, DE, FR, GB, IT, LI, LU, NL, SE

**1.** N-(substituted)-1-(piperazine alkyl) or -4-(piperidine)-α-(3,5-di-alkyl-4-hydroxyphenyl)-α,α-dialkyl acetamides having the general formula

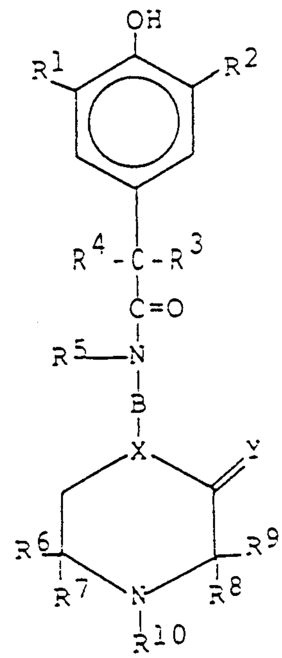

wherein $R^1$ and $R^2$ are alkyl and cycloalkyl groups containing up to 12 carbon atoms, alkylcycloalkyl groups wherein the alkyl groups contain 1 to 8 carbon atoms; $R^3$ and $R^4$ are alkyl groups containing 1 to 18 carbon atoms, $R^5$ is hydrogen, alkyl and cycloalkyl containing up to 12 carbon atoms, alkylcycloalkyl and aryl or alkaryl groups wherein the alkyl groups contain 1 to 8 carbon atoms, $R^6$, $R^7$, $R^8$ and $R^9$ are alkyl and cycloalkyl groups containing up to 12 carbon atoms, alkylcycloalkyl and aryl or alkaryl groups wherein the alkyl groups contain 1 to 8 carbon atoms, B is a bond in case X represents a -CH-group or in case X represents N, B is an alkylene group $-(CH_2)_n-$ wherein n is 1 to 8; $R^{10}$ is hydrogen or alkyl radicals containing 1 to 18 carbon atoms, O or OH; and Y is $H_2$ or O; whereby aryl represents phenyl or naphthyl.

2. N-alkyl-N-(3,3,5,5-tetraalkyl-2-keto-1-piperazine alkyl)-α-(3,5-di-alkyl-4-hydroxyphenyl-α,α-dialkylacetamides of claim 1 wherein $R^3$ and $R^4$ contain 1 to 8 carbon atoms; and $R^1$, $R^2$, $R^5$, $R^6$, $R^7$, $R^8$ and $R^9$ are alkyl groups containing 1 to 8 carbon atoms, and $R^{10}$ is an alkyl group containing 1 to 8 carbon atoms; in B n is 2 to 4.

3. The acetamides of claim 2 wherein at least one of $R^1$ and $R^2$ is t-butyl or t-amyl and $R^3$ and $R^4$ are alkyl radicals containing 1 to 4 carbon atoms.

4. N-methyl-N-(2,2,6,6-tetramethyl-4-piperidinyl)-α-(3,5-di-t-butyl-4-hydroxyphenyl)-2-methylpropionamide of claim 1.

5. N-isopropyl-N-[2-(3,3,5,5-tetramethyl-1-piperazin-2-onyl)ethyl]-α-(3,5-di-t-butyl-4-hydroxyphenyl)-2-methylpropionamide of claim 1.

6. A composition comprising (1) organic materials subject to degradation and stabilizing amounts of (2) N-(substituted)-1-(piperazine alkyl) or -4-(piperidine)-α-(3,5-di-alkyl-4-hydroxyphenyl)-α,α-dialkyl acetamides according to claims 1 to 5.

7. A process for preparing the acetamides of claim 1 comprising reacting together a 2,6-dialkylphenol having the formula

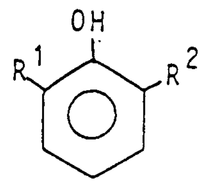

wherein $R^1$ and $R^2$ have the meanings defined in claim 1, a ketone selected from the group consisting of dialkyl ketones, wherein the alkyl groups contain 1 to 18 carbon atoms, a haloform selected from the group consisting of chloroform and bromoform, and an alkali metal hydroxide and piperidines and the piperazines, having the general formula

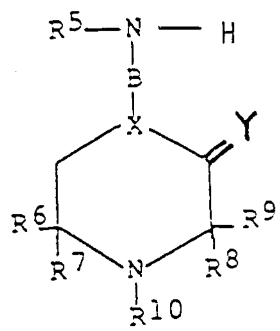

wherein $R^5$, $R^6$, $R^7$, $R^8$, $R^9$ $R^{10}$, X, Y and B have the meanings defined in claim 1.

**8.** A process of claim 7 wherein the ketone is a dialkyl ketone of the formula

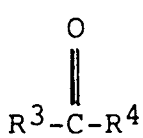

wherein $R^3$ and $R^4$ are alkyl radicals containing 1 to 8 carbon atoms, and $R^1$, $R^2$, $R^5$, $R^6$, $R^7$, $R^8$ and $R^9$ are alkyl groups containing 1 to 8 carbon atoms.

**Claims for the following Contracting State : AT**

**1.** A process for preparing N-(substituted)-1-(piperazine alkyl) or -4-(piperidine)-α-(3,5-di-alkyl-4-hydroxyphenyl)-α,α-dialkyl acetamides having the general formula

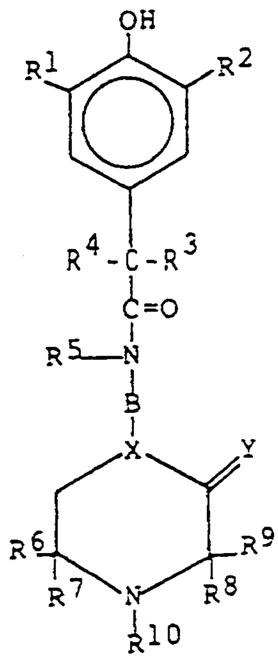

wherein $R^1$ and $R^2$ are alkyl and cycloalkyl groups containing up to 12 carbon atoms, alkylcycloalkyl groups wherein the alkyl groups contain 1 to 8 carbon atoms; $R^3$ and $R^4$ are alkyl groups containing 1 to 18 carbon atoms, $R^5$ is hydrogen, alkyl and cycloalkyl containing up to 12 carbon atoms, alkyl-cycloalkyl and aryl or alkaryl groups wherein the alkyl groups contain 1 to 8 carbon atoms, $R^6$, $R^7$, $R^8$ and $R^9$ are alkyl and cycloalkyl groups containing up to 12 carbon atoms, alkyl-cycloalkyl and aryl or alkaryl groups wherein the alkyl groups contain 1 to 8 carbon atoms, B is a bond in case X represents a -CH-group or in case X represents N, B is an alkylene group $-(CH_2)_n-$ wherein n is 1 to 8; $R^{10}$ is hydrogen or alkyl radicals containing 1 to 18 carbon atoms, O or OH; and Y is $H_2$ or O; whereby aryl represents phenyl or naphthyl, comprising reacting together a 2,6-dialkylphenol having the formula

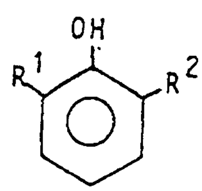

wherein $R^1$ and $R^2$ have the meanings as defined above, a ketone selected from the group consisting of dialkyl ketones of the formula

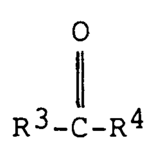

wherein the alkyl groups contain 1 to 18 carbon atoms, a haloform selected from the group consisting of chloroform and bromoform, and an alkali metal hydroxide and piperidines and the piperazines, having the general formula

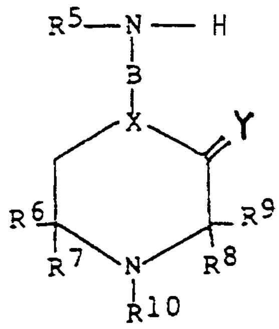

wherein $R^5$, $R^6$, $R^7$, $R^8$, $R^9$, $R^{10}$, X, Y and B have the meanings as defined above.

**2.** Process for preparing N-alkyl-N-(3,3,5,5-tetraalkyl-2-keto-1-piperazine alkyl)-α-(3,5-di-alkyl-4-hydroxyphenyl-α,α-dialkylacetamides of claim 1 wherein $R^3$ and $R^4$ contain 1 to 8 carbon atoms; and $R^1$, $R^2$, $R^5$, $R^6$, $R^7$, $R^8$ and $R^9$ are alkyl groups containing 1 to 8 carbon atoms, and $R^{10}$ is an alkyl group containing 1 to 8 carbon atoms; in B n is 2 to 4.

**3.** The process of claim 2 wherein at least one of $R^1$ and $R^2$ is t-butyl or t-amyl and $R^3$ and $R^4$ are alkyl radicals containing 1 to 4 carbon atoms.

**4.** The process for preparing N-methyl-N-(2,2,6,6-tetramethyl-4-piperidinyl)-α-(3,5-di-t-butyl-4-hydroxyphenyl)-2-methylpropionamide of claim 1.

**5.** The process for preparing N-isopropyl-N-[2-(3,3,5,5-tetramethyl-1-piperazin-2-onyl)ethyl]-α-(3,5-di-t-butyl-4-hydroxyphenyl)-2-methylpropionamide of claim 1.

**6.** A process for preparing a stabilized composition comprising (1) organic materials subject to degradation and stabilizing amounts of (2) N-(substituted)-1-(piperazine alkyl) or -4-(piperidine)-α-(3,5-dialkyl-4-hydroxyphenyl)-α,α-dialkyl acetamides prepared according to claims 1 to 5 by mixing (1) and (2).

**Patentansprüche**
**Patentansprüche für folgende Vertragsstaaten : BE, CH, DE, FR, GB, IT, LI, LU, NL, SE**

**1.** N-(Substituiert)-1-(piperazinalkyl)- oder -4-(piperidin)-α-(3,5-dialkyl-4-hydroxyphenyl)-α,α-dialkylacetamide der allgemeinen Formel

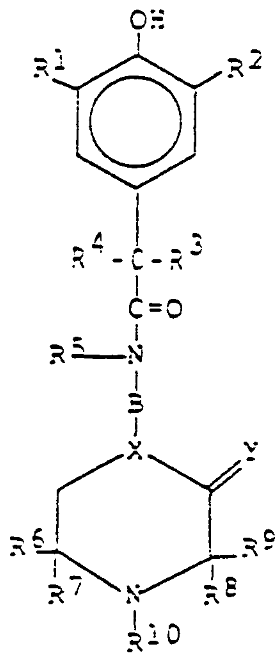

worin

| | |
|---|---|
| $R^1$ und $R^2$ | bis zu 12 Kohlenstoff-Atome enthaltende Alkyl- und Cycloalkyl-Gruppen bzw. Alkylcycloalkyl-Gruppen, worin die Alkyl-Gruppen 1 bis 8 Kohlenstoff-Atome enthalten, sind, |
| $R^3$ und $R^4$ | Alkyl-Gruppen mit 1 bis 18 Kohlenstoff-Atomen sind, |
| $R^5$ | Wasserstoff, Alkyl und Cycloalkyl mit bis zu 12 Kohlenstoff-Atomen, Alkylcyloalkyl- und Aryl- oder Alkaryl-Gruppen, worin die Alkyl-Gruppen 1 bis 8 Kohlenstoff-Atome enthalten, sind, |
| $R^6$, $R^7$, $R^8$ und $R^9$ | Alkyl- und Cycloalkyl-Gruppen mit bis zu 12 Kohlenstoff-Atomen, Alkylcyloalkyl- und Aryl- oder Alkaryl-Gruppen, worin die Alkyl-Gruppen 1 bis 8 Kohlenstoff-Atome enthalten, sind, |
| B | in dem Fall, in dem X eine -CH- Gruppe repräsentiert, eine Bindung ist und in dem Fall, in dem X N repräsentiert, eine Alkylen-Gruppe $-(CH_2)_n-$ ist, worin n 1 bis 8 ist, |
| $R^{10}$ | Wasserstoff oder ein Alkyl-Rest mit 1 bis 18 Kohlenstoff-Atomen, O oder OH ist und |
| Y | $H_2$ oder O ist, |

wobei Aryl Phenyl oder Naphthyl darstellt.

**2.** N-Alkyl-N-(3,3,5,5-tetraalkyl-2-keto-1-piperazinalkyl)-α-(3,5-dialkyl-4-hydroxyphenyl)-α,α-dialkylacetamide nach Anspruch 1, worin

| | |
|---|---|
| $R^3$ und $R^4$ | 1 bis 8 Kohlenstoff-Atome enthalten und |
| $R^1$, $R^2$, $R^5$, $R^6$, $R^7$, $R^8$ und $R^9$ | Alkyl-Gruppen mit 1 bis 8 Kohlenstoff-Atomen sind und |
| $R^{10}$ | eine Alkyl-Gruppe mit 1 bis 8 Kohlenstoff-Atomen ist und |

in B n 2 bis 4 ist.

**3.** Acetamide nach Anspruch 2, worin wenigstens eine der Gruppen $R^1$ und $R^2$ t-Butyl oder t-Amyl ist und $R^3$ und $R^4$ Alkyl-Gruppen mit 1 bis 4 Kohlenstoff-Atomen sind.

**4.** N-Methyl-N-(2,2,6,6-tetramethyl-4-piperidinyl)-α-(3,5-di-t-butyl-4-hydroxyphenyl)-2-methylpropionamid nach Anspruch 1.

**5.** N-Isopropyl-N-[2-(3,3,5,5-tetramethyl-1-piperazin-2-onyl)ethyl]-α-(3,5-di-t-butyl-4-hydroxyphenyl)-2-methylpropionamid nach Anspruch 1.

**6.** Zusammensetzung, umfassend

(1) organische Stoffe, die der Gefahr eines Abbaus unterliegen, und stabilisierende Mengen von

(2) N-(Substituiert)-1-(piperazinalkyl)- oder -4-(piperidin)-α-(3,5-dialkyl-4-hydroxyphenyl)-α,α-dialkyl-acetamiden nach den Ansprüchen 1 bis 5.

**7.** Verfahren zur Herstellung der Acetamide nach Anspruch 1, umfassend die gemeinsame Umsetzung eines 2,6-Dialkylphenols der Formel

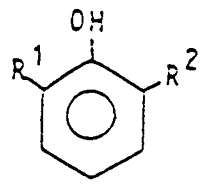

,

in der $R^1$ und $R^2$ die in Anspruch 1 angegebenen Bedeutungen haben, eines Ketons, das aus der aus Dialkylketonen, in denen die Alkyl-Gruppen 1 bis 18 Kohlenstoff-Atome enthalten, bestehenden Gruppe ausgewählt ist, eines Haloforms, das aus der aus Chloroform und Bromoform bestehenden Gruppe ausgewählt ist, und eines Alkalimetallhydroxids und von Piperidinen und Piperazinen der allgemeinen Formel

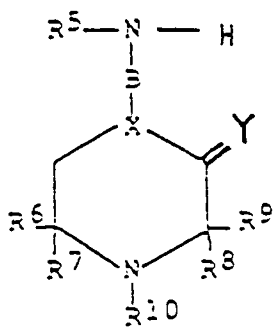

,

worin $R^5$, $R^6$, $R^7$, $R^8$, $R^9$, $R^{10}$, X, Y und B die in Anspruch 1 angegebenen Bedeutungen haben.

**8.** Verfahren nach Anspruch 7, worin das Keton ein Dialkylketon der Formel

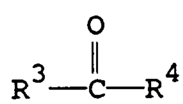

ist, worin

| | |
|---|---|
| $R^3$ und $R^4$ | Alkyl-Reste sind, die 1 bis 8 Kohlenstoff-Atome enthalten, und |
| $R^1$, $R^2$, $R^5$, $R^6$, $R^7$, $R^8$ und $R^9$ | Alkyl-Gruppen mit 1 bis 8 Kohlenstoff-Atomen sind. |

**Patentansprüche für folgenden Vertragsstaat : AT**

**1.** Verfahren zur Herstellung von N-(Substituiert)-1-(piperazinalkyl)- oder -4-(piperidin)-α-(3,5-dialkyl-4-hydroxyphenyl)-α,α-dialkyl-acetamiden der allgemeinen Formel

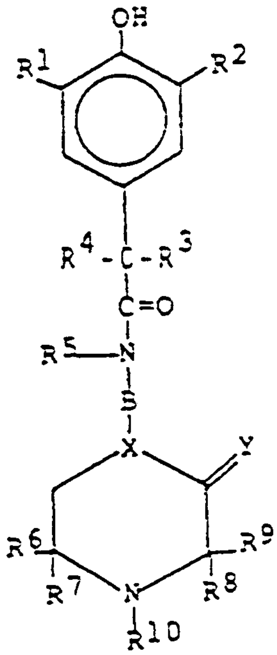

,

worin

R[1] und R[2] bis zu 12 Kohlenstoff-Atome enthaltende Alkyl- und Cycloalkyl-Gruppen bzw. Alkylcycloalkyl-Gruppen, worin die Alkyl-Gruppen 1 bis 8 Kohlenstoff-Atome enthalten, sind,

R[3] und R[4] Alkyl-Gruppen mit 1 bis 18 Kohlenstoff-Atomen sind,

R[5] Wasserstoff, Alkyl und Cycloalkyl mit bis zu 12 Kohlenstoff-Atomen, Alkylcyloalkyl- und Aryl- oder Alkaryl-Gruppen, worin die Alkyl-Gruppen 1 bis 8 Kohlenstoff-Atome enthalten, sind,

R[6], R[7], R[8] und R[9] Alkyl- und Cycloalkyl-Gruppen mit bis zu 12 Kohlenstoff-Atomen, Alkylcyloalkyl- und Aryl- oder Alkaryl-Gruppen, worin die Alkyl-Gruppen 1 bis 8 Kohlenstoff-Atome enthalten, sind,

B in dem Fall, in dem X eine -CH- Gruppe repräsentiert, eine Bindung ist und in dem Fall, in dem X N repräsentiert, eine Alkylen-Gruppe $-(CH_2)_n-$ ist, worin n 1 bis 8 ist,

R[10] Wasserstoff oder ein Alkyl-Rest mit 1 bis 18 Kohlenstoff-Atomen, O oder OH ist und

Y $H_2$ oder O ist,

wobei Aryl Phenyl oder Naphthyl darstellt,
umfassend
die Umsetzung eines 2,6-Dialkylphenols der Formel

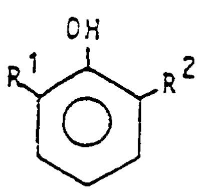

,

in der R[1] und R[2] die im vorstehenden angegebenen Bedeutungen haben,
eines Ketons, das aus der aus Dialkylketonen der Formel

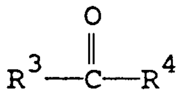

,

in denen die Alkyl-Gruppen 1 bis 18 Kohlenstoff-Atome enthalten, bestehenden Gruppe ausgewählt ist, eines Haloforms, das aus der aus Chloroform und Bromoform bestehenden Gruppe ausgewählt ist, und eines Alkalimetallhydroxids und von Piperidinen und Piperazinen der allgemeinen Formel

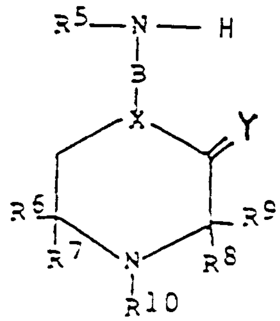

,

worin $R^5$, $R^6$, $R^7$, $R^8$, $R^9$, $R^{10}$, X, Y und B die im vorstehenden angegebenen Bedeutungen haben.

**2.** Verfahren zur Herstellung von N-Alkyl-N-(3,3,5,5-tetraalkyl-2-keto-1-piperazinalkyl)-α-(3,5-dialkyl-4-hydroxyphenyl)-α,α-dialkylacetamide nach Anspruch 1, worin

| | |
|---|---|
| $R^3$ und $R^4$ | 1 bis 8 Kohlenstoff-Atome enthalten und |
| $R^1$, $R^2$, $R^5$, $R^6$, $R^7$, $R^8$ und $R^9$ | Alkyl-Gruppen mit 1 bis 8 Kohlenstoff-Atomen sind und |
| $R^{10}$ | eine Alkyl-Gruppe mit 1 bis 8 Kohlenstoff-Atomen ist und |

in B n 2 bis 4 ist.

**3.** Verfahren nach Anspruch 2, worin wenigstens eine der Gruppen $R^1$ und $R^2$ t-Butyl oder t-Amyl ist und $R^3$ und $R^4$ Alkyl-Reste mit 1 bis 4 Kohlenstoff-Atomen sind.

**4.** Verfahren zur Herstellung von N-Methyl-N-(2,2,6,6-tetramethyl-4-piperidinyl)-α-(3,5-di-t-butyl-4-hydroxyphenyl)-2-methylpropionamid nach Anspruch 1.

**5.** Verfahren zur Herstellung von N-Isopropyl-N-[2-(3,3,5,5-tetramethyl-1-piperazin-2-onyl)ethyl]-α-(3,5-di-t-butyl-4-hydroxyphenyl)-2-methylpropionamid nach Anspruch 1.

**6.** Verfahren zur Herstellung einer stabilisierten Zusammensetzung, umfassend
(1) organische Stoffe, die der Gefahr eines Abbaus unterliegen, und stabilisierende Mengen von
(2) N-(Substituiert)-1-(piperazinalkyl)- oder -4-(piperidin)-α-(3,5-dialkyl-4-hydroxyphenyl)-α,α-dialkylacetamiden, die nach den Ansprüchen 1 bis 5 hergestellt sind,
durch Vermischen von (1) und (2).

## Revendications

## Revendications pour les Etats contractants suivants : BE, CH, DE, FR, GB, IT, LI, LU, NL, SE

**1.** 1-(pipérazine-alkyl)- ou 4-(pipéridine)-α-(3,5-dialkyl-4-hydroxyphényl)-α,α-dialkyl-acétamides N-substitués ayant la formule générale

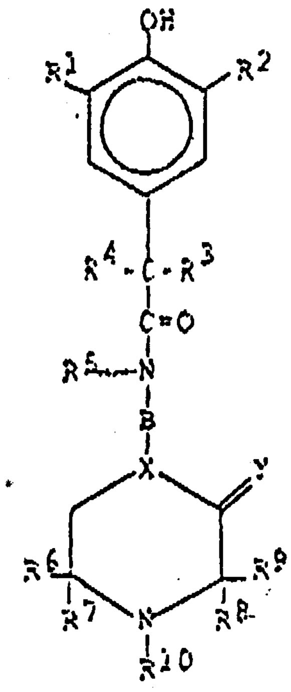

dans laquelle $R^1$ et $R^2$ sont des groupes alkyle et cycloalkyle contenant jusqu'à 12 atomes de carbone, des groupes alkylcycloalkyle dont les groupes alkyle contiennent de 1 à 8 atomes de carbone ; $R^3$ et $R^4$ sont des groupes alkyle contenant de 1 à 18 atomes de carbone, $R^5$ est un hydrogène ou un radical alkyle ou cycloalkyle contenant jusqu'à 12 atomes de carbone, alkylcycloalkyle, aryle et alkylaryle , où le groupe alkyle contient de 1 à 8 atomes de carbone, $R^6$, $R^7$, $R^8$ et $R^9$ sont des radicaux alkyle et cycloalkyle contenant jusqu'à 12 atomes de carbone, alkylcycloalkyle et aryle ou alkylaryle où les groupes alkyle contiennent de 1 à 8 atomes de carbone, B est une liaison quand X représente un groupe -CH- ou quand X représente N, B est un groupe alkylène $-(CH_2)_n-$ où n vaut de 1 à 8 ; $R^{10}$ est un hydrogène ou un radical alkyle contenant de 1 à 18 atomes de carbone, O ou OH ; et Y est $H_2$ ou O ; aryle représentant les radicaux phényle ou naphtyle.

**2.** N-alkyl-N-(3,3,5,5-tétraalkyl-2-céto-1-pipérazine alkyl)-α-(3,5-dialkyl-4-hydroxyphényl-α,α-dialkylacétamides selon la revendication 1, où $R^3$ et $R^4$ contiennent de 1 à 8 atomes de carbone ; et $R^1$, $R^2$, $R^5$, $R^6$, $R^7$, $R^8$ et $R^9$ sont des groupes alkyle contenant de 1 à 8 atomes de carbone, et $R^{10}$ est un groupe alkyle contenant de 1 à 8 atomes de carbone ; dans B, n vaut de 2 à 4.

**3.** Acétamides selon la revendication 2, où au moins l'un des radicaux $R^1$ et $R^2$ est le radical tert-butyle ou tert-amyle, et $R^3$ et $R^4$ sont des radicaux alkyle contenant de 1 à 4 atomes de carbone.

**4.** N-méthyl-N-(2,2,6,6-tétraméthyl-4-pipéridinyl)-α-(3,5-di-tert-butyl-4-hydroxyphényl)-2-méthylpropionamide salon la revendication 1.

**5.** N-isopropyl-N-[2-(3,3,5,5-tétraméthyl-1-pipérazine-2-onyl)éthyl]-α-(3,5-di-tert-butyl-4-hydroxyphényl)-2-méthylpropionamide selon la revendication 1.

**6.** Composition comprenant (1) des composé, organiques soumis à dégradation et des quantités à effet de stabilisation des (2) 1-(pipérazine-alkyl)- ou 4-(pipéridine)-α-(3,5-dialkyl-4-hydroxyphényl)-α,α-dialkylacétamides N-substitués selon les revendications 1 à 5.

**7.** Procédé de préparation des acétamides selon la revendication 1, qui consiste à faire réagir ensemble un 2,6-dialkylphénol ayant la formule :

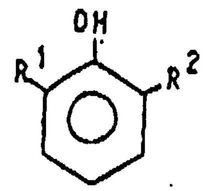

dans laquelle $R^1$ et $R^2$ ont les significations données dans la revendication 1, une cétone choisie parmi l'ensemble comprenant les dialkylcétones, les groupes alkyle contenant de 1 à 18 atomes de carbone, un halogénoforme choisi parmi l'ensemble comprenant le chloroforme et le bromoforme, et un hydroxyde d'un métal alcalin et des pipéridines, et les pipérazines ayant la formule générale :

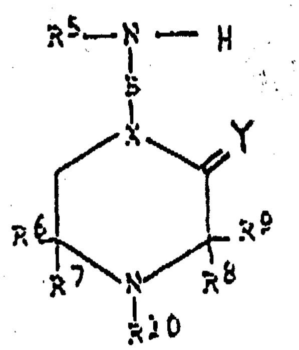

où $R^5$, $R^6$, $R^7$, $R^8$, $R^9$, $R^{10}$, X, Y et B ont les significations données dans la revendication 1.

**8.** Procédé selon la revendication 7, dans lequel la cétone est une dialkylcétone de formule

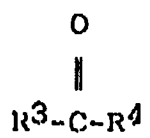

dans laquelle $R^3$ et $R^4$ sont des radicaux alkyle ayant de 1 à 8 atomes de carbone, et $R^1$, $R^2$, $R^5$, $R^6$, $R^7$, $R^8$ et $R^9$ sont des groupes alkyle ayant de 1 à 8 atomes de carbone.

**Revendications pour l'Etat contractant suivant : AT**

**1.** Procédé de préparation des 1-(pipérazine-alkyl)- ou 4-(pipéridine)-α-(3,5-dialkyl-4-hydroxyphényl)-α,α-dialkyl-acétamides N-substitués ayant la formule générale

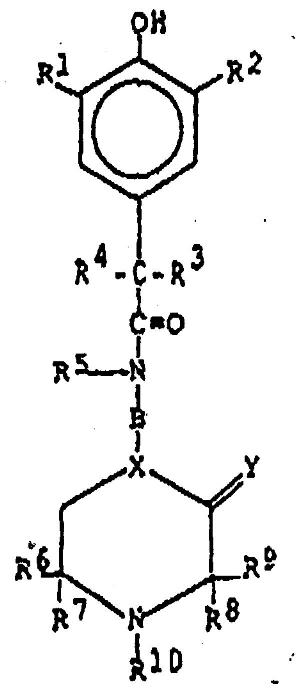

dans laquelle $R^1$ et $R^2$ sont des groupes alkyle et cycloalkyle contenant jusqu'à 12 atomes de carbone, des groupes alkylcycloalkyle dont les groupes alkyle contiennent de 1 à 8 atomes de carbone ; $R^3$ et $R^4$ sont des groupes alkyle contenant de 1 à 18 atomes de carbone, $R^5$ est un hydrogène un radical alkyle et cycloalkyle contenant jusqu'à 12 atomes de carbone, alkylcycloalkyle, et aryle ou alkylaryle, où les groupes alkyle contiennent de 1 à 8 atomes de carbone, $R^6$, $R^7$, $R^8$ et $R^9$ sont des radicaux alkyle ou cycloalkyle contenant jusqu'à 12 atomes de carbone, alkylcycloalkyle et aryle ou alkylaryle où les groupes alkyle contiennent de 1 à 8 atomes de carbone, B est une liaison quand X représente un groupe -CH- ou quand X représente N, B est un groupe alkylène $-(CH_2)_n-$ où n vaut de 1 à 8 ; $R^{10}$ est un hydrogène ou un radical alkyle contenant de 1 à 18 atomes de carbone, O ou OH ; et Y est $H_2$ ou O ; aryle représentant les radicaux phényle ou naphtyle, qui consiste à faire réagir ensemble un 2,6-dialkylphénol ayant la formule

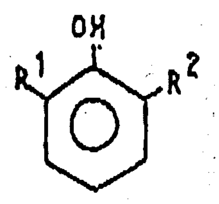

dans laquelle $R^1$ et $R^2$ ont les significations données ci-dessus, une cétone choisie parmi l'ensemble comprenant les dialkylcétones de formule

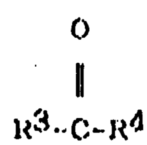

où les groupes alkyle contiennent de 1 à 18 atomes de carbone, un halogénoforme choisi parmi l'ensemble comprenant le chloroforme et le bromoforme, et un hydroxyde d'un métal alcalin et des pipéridines et les pipérazines ayant la formule générale

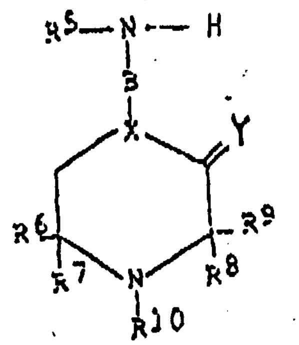

où $R^5$, $R^6$, $R^7$, $R^8$, $R^9$, $R^{10}$, X, Y et B ont les significations données ci-dessus.

2. Procédé de préparation des N-alkyl-N-(3,3,5,5-tétraalkyl-2-céto-1-pipérazine alkyl)-α-(3,5-dialkyl-4-hydroxyphényl-α,α-dialkylacétamides selon la revendication 1, où $R^3$ et $R^4$ contiennent de 1 à 8 atomes de carbone ; et $R^1$, $R^2$, $R^5$, $R^6$, $R^7$, $R^8$ et $R^9$ sont des groupes alkyle contenant de 1 à 8 atomes de carbone, et $R^{10}$ est un groupe alkyle contenant de 1 à 8 atomes de carbone ; dans B, n vaut de 2 à 4.

3. Procédé selon la revendication 2, où au moins l'un des radicaux $R^1$ et $R^2$ est le radical tert-butyle ou tert-amyle, et $R^3$ et $R^4$ sont des radicaux alkyle contenant de 1 à 4 atomes de carbone.

4. Procédé de préparation du N-méthyl-N-(2,2,6,6-tétraméthyl-4-pipéridinyl)-α-(3,5-di-tert-butyl-4-hydroxyphényl)-2-méthylpropionamide selon la revendication 1.

5. Procédé de préparation du N-isopropyl-N-[2-(3,3,5,5-tétraméthyl-1-pipérazine-2-onyl)éthyl]-α-(3,5-di-tert-butyl-4-hydroxyphényl)-2-méthylpropionamide selon la revendication 1.

6. Procédé de préparation d'une composition stabilisée comprenant (1) des composés organiques soumis à dégradation et des quantités à effet de stabilisation des (2) 1-(pipérazine-alkyl)- ou 4-(pipéridine)-α-(3,5-dialkyl-4-hydroxyphényl)-α,α-dialkyl-acétamides N-substitués selon les revendications 1 à 5, par mélange de (1) et de (2).